# EUROPEAN PATENT SPECIFICATION

(11) **EP 1 830 857 B1**
(45) Date of publication and mention of the grant of the patent: **15.09.2010**
(21) Application number: 05856121.8
(22) Date of filing: 30.12.2005
(51) Int. Cl.: A61K 31/573, A61K 9/20, A61K 9/28, A61P 37/06

(54) **BECLOMETHASONE DIPROPIONATE AND PREDNISONE FOR REDUCING MORTALITY ASSOCIATED WITH GRAFT-VERSUS-HOST DISEASE**
BECLOMETHASONDIPROPIONAT UND PREDNISON ZUR VERMINDERUNG DER STERBLICHKEIT IN ZUSAMMENHANG MIT DER GRAFT-VERSUS-HOST-REAKTION
LE DIPROPRIONATE DE BECLOMETHASONE ET LA PREDNISONE POUR REDUIRE LA MORTALITE ASSOCIEE A LA REACTION DU GREFFON CONTRE L'HOTE

(30) Priority: 30.12.2004 US 640178 P
(43) Date of publication of application: 12.09.2007
(73) Proprietor: Soligenix, Inc., Miami, FL 33131 (US)
(72) Inventor: McDONALD, George B., Bellevue, WA 98004 (US); STERGIS, Nicholas, Ewing, NJ 08628 (US); KANZER, Steve, Miami Beach, FL 33139 (US)
(74) Representative: Winter, Brandl, Fürniss, Hübner, Röss, Kaiser, Polte - Partnerschaft
(86) International application number: PCT/US2005/047666
(87) International publication number: WO 2006/072093

(56) References cited:
- US-A- 5 968 912
- US-A- 6 096 728
- US-A1- 2002 086 857
- US-A1- 2003 032 631
- BAEHR, P. H. ET AL: "Oral beclomethasone dipropionate for treatment of human intestinal graft - versus -host disease" TRANSPLANTATION, vol. 60, no. 11, 15 December 1995 (1995-12-15), pages 1231-1238, XP009094484 ISSN: 0041-1337
- MCDONALD ET AL: "Oral beclomethasone dipropionate for treatment of intestinal graft-versus-host disease: A randomized, controlled trial" GASTROENTEROLOGY, ELSEVIER, PHILADELPHIA, PA, US, vol. 115, no. 1, July 1998 (1998-07), pages 28-35, XP005138977 ISSN: 0016-5085
- DOR BIOPHARMA ET AL: "DOR biopharma announces the completion of the pivotal phase III clinical trial of orbec" INTERNET CITATION, [Online] 15 September 2004 (2004-09-15), XP007903805 Retrieved from the Internet: URL:http://info dorbiopharma.com> [retrieved on 2008-01-10] -& DATABASE INTEGRITY [Online] Prous Science; DOR Biopharma Press Release 15 September 2004 (2004-09-15), "DOR biopharma announces the completion of the pivotal phase III clinical trial of orbec" XP007903807 retrieved from HTTP://INTEGRITY.PROUS.COM Database accession no. 91480
- DOR BIOPHARMA INC: "DOR biopharma, inc. announces top-line results of pivotal phase III clinicaltrial of orbec for intestinal graft-versus-host disease orbec demonstrates highly statistically significant reduction in mortality and positive trend in primary endpoint" INTERNET CITATION, [Online] 30 December 2004 (2004-12-30), XP007903806 Retrieved from the Internet: URL:http://info dorbiopharma.com/> [retrieved on 2008-01-10]
- MCDONALD, G. B.: "Oral beclomethasone dipropionate: a topically active corticosteroid for the treatment of gastrointestinal graft - versus host disease following allogeneic hematopoietic cell transplantation" EXPERT OPINION ON INVESTIGATIONAL DRUGS, vol. 16, no. 10, 2007, pages 1709-1724, XP007903819 ISSN: 1354-3784

## Description

### Field of the Invention

Treatment of gastrointestinal Graft-Versus-Host Disease (GVHD) by an orally effective therapeutic agent according to claim 1.

### Background of the Invention

Hematopoietic cell transplantation encompasses bone marrow transplantation, peripheral blood stem cell transplantation, umbilical vein blood transplantation, or any other source of pleuripotent hematopoietic stem cells. It often gives rise to a complication known as graft-versus-host disease some days or weeks after the cell transplant.

The standard treatment of acute graft-versus-host disease (GVHD) is oral or IV corticosteroids, usually prednisone at a dose of 2 mg/kg/day (Sullivan KM. 1999. Graft-versus-host disease. Hematopoietic Cell Transplantation, ed. Thomas, E. D:, Forman, S. J., and Blume, K. G.,2nd: 515-36. Cambridge, MA: Blackwell Scientific. 2nd ed.). For patients who progress to Grade III or IV GVHD, this dose of prednisone is frequently required for many weeks, even when other immunosuppressive drugs are added to the treatment regimen. For patients who present with symptoms of gastrointestinal (GI) GVHD that are less severe, prednisone is dosed at 2 mg/kg/day for a shorter period of time, usually 1-3 weeks, depending on response, after which the dose is tapered to avoid corticosteroid side effects (Weisdorf DJ, Snover DC, Haake R, Miller WJ, McGlave PB et al. 1990. Acute upper gastrointestinal graft-versus-host disease: clinical significance and response to immunosuppressive therapy. Blood 76: 624-629; Wu D, Hockenbery DM, Brentnall TA, Baehr PH, Ponec RJ et al. 1998. Persistent nausea and anorexia after marrow transplantation: a prospective study of 78 patients. Transplantation 66: 1319-1324).

Document US 2003/032631 A1 describes a method for the treatment for blood-bome cancers by administering an effective amount of a topically active corticosteroid, e.g. BDP in combination with the systemically active corticosteroid prednisone to a patient who has undergone hematopoietic cell transplantation to prevent or reduce symptoms of graft-versus-host-disease while maintaining a graft-versus-leukemia reaction effective to eliminate or reduce the number of cancer cells. The administration, according to the prior art of US 2003/032631 A1, preferably begins at day 1 following hematopoietic cell transplantation and preferably continues up to day 80 following hematopoietic cell transplantation.

Recurrence of GI symptoms is frequent but not easy to predict in individual patients. Patients who experience a worsening of symptoms during or following prednisone taper are retreated with high doses of corticosteroids, and generally respond. The frequency of response of GI symptoms after second and third courses of corticosteroids is 80-90%, but each course of treatment is for a minimum of 1-3 weeks,

followed by tapering of the prednisone to allow recovery of the hypothalamic-pituitary-adrenal (HPA) axis (Strasser SI and McDonald GB. 1999. Gastrointestinal and hepatic complications in Hematopoietic Cell Transplantation , ed. Thomas, E. D., Blume, K. G., and Forman, S. J., 2nd: 627-58. Cambridge, MA: Blackwell Scientific Publications. 2nd ed.). The side effects of prolonged high dose corticosteroid treatment are well known, and include hyperglycemia, hypertension, neuropsychiatric symptoms, muscle weakness, infections, bone demineralization, and body habitus changes (cushingoid features, including moon facies, buffalo hump, and thinning and striae of the skin).

Clearly, alternatives to corticosteroid therapy with comparable efficacy and less toxicity are desirable in order to increase the usefulness of hematopoietic cell transplantation. Beclomethasone 17,21-dipropionate (BDP) is a synthetic diester of beclomethasone, a corticosteroid analog that has appeal in the treatment of GI GVHD by virtue of its ability to direct therapy to inflamed GI mucosa. BDP in capsule form at a dose of 8 mg/day divided into 4 equal doses has been shown to be well tolerated and to provide clinical benefit in the treatment of Grade II GI GVHD (Baehr PH, Levine DS, Bouvier ME, Hockenberry DM, Gooley TA et al. 1995. Oral beclomethasone dipropionate for treatment of human intestinal graft-versus-host disease. Transplantation 60: 1231-1238; McDonald GB, Bouvier M, Hockenbery DM, Stern JM, Gooley T et al. 1998. Oral beclomethasone dipropionate for treatment of intestinal graft-versus-host disease: a randomized, controlled trial. Gastroenterology 115: 28-35).

While significant advances have been made with regard to the treatment of GVHD following bone marrow transplantation, there is still a need in the art for improved methods, particularly with respect to reducing mortality from both the intestinal mucosal damage associated with GVHD and the underlying disease, such as leukemia, which gave rise to the need for the hematopoietic cell transplantation. One obstacle to progress in this field has been the difficulty of identifying whether a patient's symptoms were due to GVHD, the immunosurpressive methods used to prepare the patient for the transplant, or due to the underlying disease. As a result many of the participants in prior studies may not have actually had GVHD or may have had a less severe case than was thought. This situation made it very difficult to evaluate any given treatment, and, in particular, whether extending the period of treatment would improve the mortality results. By careful evaluation of the GVHD status of the patients, the present invention demonstrates that extended therapy with BDP after limited high dose co-administration of prednisone significantly reduces mortality in transplant patients after treatment with the drugs for 50 days.

Leukemia, lymphoma and myeloma are cancers that originate in the bone marrow (in the case of leukemia and myeloma) or in lymphatic tissues (in the case of lymphoma). Leukemia, lymphoma and myeloma are considered to be related cancers, because they involve the uncontrolled growth of cells having similar functions and origins. The diseases result from an acquired (i.e., not inherited) genetic injury to the DNA of a single cell, which becomes abnormal (malignant) and multiplies continuously. The accumulation of malignant cells interferes with the body's production of healthy blood cells and makes the body unable to protect itself against infections.

Treatment of leukemia, lymphoma and myeloma usually involves one or more forms of chemotherapy and/or radiation therapy. These treatments destroy the malignant cells, but also destroy the body's healthy blood cells as well. Allogeneic bone marrow transplantation (BMT) is an effective therapy for the treatment of many hematologic malignancies. In allogeneic BMT, bone marrow (or, in some cases, peripheral blood) from an unrelated or a related (but not identical twin) donor is used to replace the healthy blood cells in the cancer patient. The bone marrow (or peripheral blood) contains stem cells, which are the precursors to all the different cell types (e.g., red cells, phagocytes, platelets and lymphocytes) found in blood. Allogeneic BMT has both a restorative effect and a curative effect. The restorative effect arises from the ability of the stem cells to repopulate the cellular components of blood. The curative properties of allogeneic BMT derive largely from a graft-versus-leukemia (GVL) effect. The hematopoietic cells from the donor (specifically, the T lymphocytes) attack the cancerous cells, enhancing the suppressive effects of the other forms of treatment. Essentially, the GVL effect comprises an attack on the residual tumor cells by the blood cells derived from the BMT, making it less likely that the malignancy will return after transplant. Controlling the GVL effect prevents escalation of the GVL effect into GVHD. A similar effect against tumors (GVT) is also available to exploit in this way.

BMT, while originally referring specifically to bone marrow transplantation, more recently has become a generic term for blood or marrow transplantation, to encompass the use of blood transplantation as well as bone marrow transplantation. In many cases, the more specific term "stem cell transplantation" (or SCT) is now used.

Allogeneic BMT is often toxic to the patient. The toxicity arises from the difficulty in dissociating the GVL or GVT effect from graft-versus-host disease (GVHD), an often-lethal complication of allogeneic BMT. Graft-versus-host disease (GVHD) is a complication of allogeneic hematopoietic cell transplantation in which tissues of the host, most frequently the skin, liver and intestine, are damaged by lymphocytes from the donor graft. When a patient has GVHD, treatment is successful only 50-75% of the time; the remainder of the patients generally die. The risk and severity of this immune-mediated condition are directly related to the degree of mismatch between a host and the donor of hematopoietic cells. For example, GVHD develops in up to 30% of recipients of human leukocyte antigen (HLA)-matched sibling marrow, in up to 60% of recipients of HLA-matched unrelated donor marrow, and in a higher percentage of recipient of HLA-mismatched marrow. Patients with mild intestinal GVHD present with anorexia, nausea, vomiting, abdominal pain and diarrhea, whereas patients with severe GVHD are disabled by these symptoms. If untreated, symptoms of intestinal GVHD persist and often progress; spontaneous remissions are unusual. In its most severe form, GVHD leads to necrosis and exfoliation of most of the epithelial cells of the intestinal mucosa, a frequently fatal condition.

U.S. Patent No. 6,096,731 (McDonald) describes a method for the treatment of GVHD or that comprises administration of a prophylactically effective amount of a topically active corticosteroid (TAC) to a patient following intestinal or liver transplantation. The TAC is administered for a period of time effective to prior to presentation of symptoms associated with either GVHD or HVGD. However, no information was given relating to methods of treatment of cancer by controlling a GVL reaction.

While significant advances have been made with regard to the treatment of GVHD following bone marrow transplantation, there is still a need in the art for improved methods for treating cancers by controlling the GVL effect and preventing the damage associated with the onset of GVHD.

### Summary of the Invention

An object of the invention is to provide a method for reducing mortality associated with GVHD by treating the patent with an oral BDP regimen that involves coadministration of: 1) a high dose of prednisone (about 1-2 mg/kg/day) for about 10 days, which is then tapered rapidly over the following 7 days to a physiological replacement dose of about 0.0625 mg/kg/day for the remainder of the treatment, and 2) about 4 - 12 mg oral BDP q.i.d. for about 50 days, where the BDP is administered in both immediate release and enteric coated preparations. A significant reduction in patient mortality is observed 200 days after the start of the treatment.

### Brief Description of the Figures

Fig. 1: Prednisone and Study Drug Dosing
Fig. 2: Cumulative treatment failures during the first 50 days in patients receiving placebo or BDP

Fig. 3: Duration of overall survival - post-randomization (safety population)

### Detailed Description of the Invention

Beclomethasone dipropionate may be formulated for oral administration by techniques well known in the formulation field, including formulation as a capsule, pill, coated microsphere with specific dissolution qualities, or emulsion. Suitable capsules or pills generally contain from 1 mg to 2 mg BDP, and typically about 1 mg BDP, plus optional fillers, such as lactose, and may be coated with a variety of materials, such as cellulose acetate phthalate.

Such capsules, microspheres or pills may be made to dissolve within various location of the intestinal tract. For example, enteric coated capsules prepared with a coating of cellulose acetate phthalate are known to dissolve in the alkaline environment of the small bowel, thus delivering its content to the small bowl and colon. Emulsion containing BDP may also be employed for oral delivery, including optional emulsifying agents.

In addition to the BDP, pharmaceutically acceptable carriers and/or diluents may be employed and are familiar to those skilled in the art. Formulations in the form of pills, capsules, microspheres, granules or tablets may contain, in addition to BDP, diluents, dispersing and surface active agents, binders and lubricants. One skilled in the art may further formulate BDP in an appropriate manner, and in accordance with accepted practices, such as those disclosed in Remington's Pharmaceutical Sciences, Gennaro, Ed., Mack Publishing Co., Easton, Pa., 1990.

In the practice of this invention the dose of BDP will generally range from 4 mg/day to 12 mg/day, and more typically range from 6 mg/day to 8 mg/day.

An important aspect of this invention is that the BDP is orally administered such that it is topically administered to the intestinal. Thus, oral administration, as that term is used herein, is not intended to encompass systemic administration, such as by intravenous injection. Rather, the BDP has little systemic availability, but high topical activity on intestinal tissue. Such limited distribution results in fewer side effects, which is a significant advantage of this invention.

In addition to differences with regard to location and timing of administration, there is also a biological basis between short-term and long term treatment regiments. Corticosteriods have been used in the acute or short term management of GVHD. Conversely, long term therapy using corticosteroids is not appropriate due to the systemic side effects of compounds such as prednisone. Therefore, co-administration of prednisone and BDP at the beginning of the treament, followed by rapid tapering of the prednisone and long term BDP therapy, is better able to reduce the mortality of GVHD the associated underlying diseases, with minimal use of prednisone.

The BDP is administered in at least two separate dosage forms, including at least one each of an immediate release and an enteric coated composition. An "immediate release" formulation is one that is intended and formulated to dissolve and have activity in the in the initial portions of the gastrointestinal tract, such as the stomach, the duodenum and the proximal small bowel.

An "enteric coated" formulation is one that is intended and formulated to dissolve and have activity in the lower intestinal tract, such as the distal small intestine, the ileocecal region or colon (large intestine). The two separate dosage forms may contain equal or different amounts of the topically active corticosteroid. The two separate dosage forms may also contain prednisone,

The preferred dosage level of the topically active corticosteroid will generally range from 2.0 mg/day to 12 mg/day, and more typically range from 2 mg/day to 4 mg/day.

The dosage level should be such that the drug does not enter the systemic circulation to any significant extent, e.g., in amounts high enough to cause adverse systemic effects, and hence these effects resulting from their presence in the systemic circulation are avoided. Alternatively, the dosage level should be such that the amount that crosses the wall of the gastrointestinal tract is less than that required to induce the undesirable side effects associated with BDP.

It is preferable to limit the number of separate doses to a small number. One, two, three or four separate doses per day are preferred.

In general, when delivered using two or more different dosage forms, the patient receives the topically active corticosteroid throughout the entire gastrointestinal tract, from the stomach to the rectum. For example, one dosage form may be formulated as a gelcapsule, and the second dosage form may be formulated as an enteric coated gelcapsule.

In one embodiment of the invention, the separate dosage forms are administered to a patient as separate tablets, pills, troches and gelcaps. In this embodiment, the separate dosage forms are designed to contain a first dosage form containing BDP that releases in the stomach and a second dosage form containing BDP that releases in the intestine, such that the simultaneous administration of the two dosage forms results in a combination of quick release and slow release of the topical corticosteroid.

In yet another preferred embodiment of the invention, the separate dosage forms are combined in a single formulation form, i.e., a single tablet or a single gelcap, for oral administration to a patient. In this embodiment of the invention, the BDP may be formulated in microspheres, polymer microspheres, hydrogels, water-in-oil emulsions, oil-in-water emulsions, liposomes, micelles, or reverse micelles to control the release in the small intestine, and separately the BDP is added external to the microspheres or drug delivery vehicle in a suitable matrix to allow for rapid release. In other words, such a formulation would contain an inner core containing, for example, an enteric coated formulation of the BDP, and an outer shell surrounding the core and containing a rapid release formulation of the drug. Note also that one or the other of the inner core or outer shell could optionally contain a second drug different from the topical corticosteroid, such as an immunosurpresser, e.g. prednisone. Such a combination results in a single formulation with both slow release and quick release characteristics.

Suitable polymeric systems for entrapment of topical steroids in microspheres, hydrogels, and nanospheres include single component polymer systems and combinations of polyalkylene oxide homopolymers, polyethylene glycol, polypropylene glycols, polyoxyethylenated polyols, polyols, polyimines, polypeptides, polyglutamic acid, polylysine, polyaspartic acid, polyacid esters, polyacrylic acid, alginate, hyaluronic acid, chitosan, carboxymethyl cellulose, hydroxypropylmethyl cellulose oligosaccharides, polysaccharides, carageenan and salts thereof, dextran, deacetylated chitosan, gelatin, block co-polymers, block copolymers of polyoxyethylene and polyoxypropylene, methoxy-PEG, methoxy-PEG amine, polyacrylyl amides, polyvinyl pyrollidones, poly lactic, polyglycolic acid, polyvinyl alcohols, and copolymers thereof.

BDP is available from a number of commercial sources, such as Schering-Plough Corporation (Kenilworth, N.J.) in bulk crystalline form. Beclomethasone 17,21-dipropionate has the following structure:

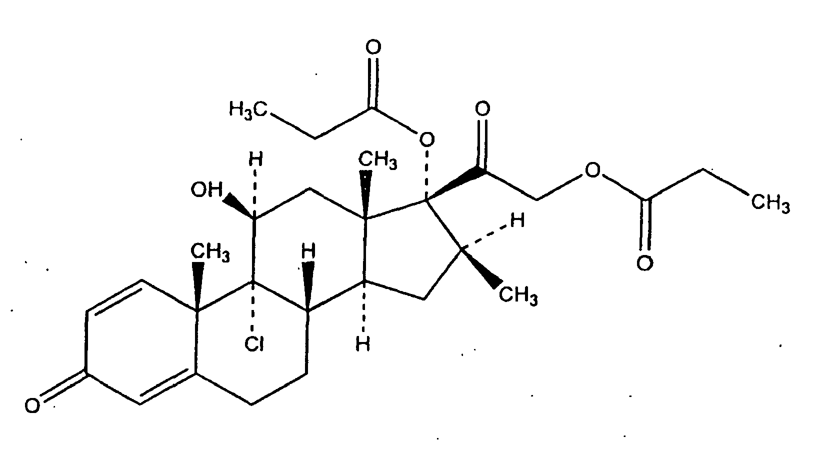

Suitable capsules or pills generally contain from 0.1 mg to 8 mg of the topically active corticosteroid, and typically about 2 mg, plus optional fillers, including binders, such as microcrystalline cellulose, gum tragacanth or gelatin; fillers, such as starch or lactose; disintegrating agents, such as con starch or alginic acid; sweetening agents, such as sucrose, saccharin or phenylalanine; or flavoring agents, such as peppermint, lemon, cinnamon, methyl salicylate, or orange flavoring. In addition, the capsules or pills may be coated with a variety of materials, such as sugars, shellacs or cellulose acetate phthalate. By adding an appropriate coating, the capsules, microspheres or pills may be made to dissolve within various location of the intestinal tract.

For example, enteric coated capsules prepared with a coating of cellulose acetate phthalate are known to remain intact in the stomach and dissolve in the alkaline environment of the small bowel, thus delivering its content to the small bowl and colon. Other useful enteric coatings may include hydroxypropyl methylcellulose phthalate, polyvinyl acetate phthalate, and those polymers based on methacrylic acid-methacrylic acid ester copolymers with acidic ionizable groups known to those in the art under the trade name "Eudragit" (Roehm GmbH & Co., Darmstadt, Germany).

The BDP may also be formulated as an oil-in-water emulsion, a water-in-oil emulsion, a multiple emulsion, (w/o/w), entrapped or associated with liposomes, and/or organized lipid phases. Lipid-based delivery systems such as emulsion systems, microemulsions systems, or lipid particulate systems are based on the use of polar lipids and related amphiphilic surfactant molecules to control the interaction of hydrophobic molecules with water. Delivery systems for hydrophobic drugs may also required the inclusion of organic solvents that are water miscible in order to increase the molecular interactions between drugs and lipid or surfactant components.

### 5.2.2 Clinical Studies with Oral BDP for the Treatment of GVHD

Oral BDP has been studied in patients with gastrointestinal manifestations of GVHD at Fred Hutchinson Cancer Research Center (FHCRC) for several years. Two studies were conducted under an investigator Investigational New Drug application (IND) in the 1990s to study the safety and efficacy of oral BDP in patients with GVHD with primarily gastrointestinal symptoms. These two studies are listed in Table 1, and each is summarized below.

**Table 1: Clinical Studies with Oral BDP**

| **Section** | **Study Title and Reference** | **Design** | **Patients** | **N** | **BDP Dosage Regimen** |
|---|---|---|---|---|---|
| A | Oral Beclomethasone Dipropionate for the Treatment of Patients with Gastrointestinal Graft-Versus-Host Disease (Study No. 615) (Baehr et al. 1995) | Phase 1/2 open-label uncontrolled | gastrointestinal GVHD Patients receiving only prophylactic immunosuppressive therapy | 23 | 8 mg/day [two 1 mg capsules (one IR and one ER) q.i.d.] for 28 days |
| | | | gastrointestinal GVHD patients receiving prednisone (0.25 - 4 mg/kg) | 17 | |
| B | Controlled Study of Prednisone With or Without Oral Beclomethasone Dipropionate for the Initial Treatment of Patients with Intestinal Graft-Versus-Host Disease (Study No. 875) (McDonald et al. 1998) | Phase 2 stratified, randomized, double-blind, placebo- controlled | gastrointestinal GVHD II patients receiving 1 mg/kg/day of prednisone for 10 | 31 | 8 mg/day BDP [two 1 mg capsules (one IR and one EC) q.i.d.] for 30 days |
| | | | days (dose was rapidly tapered starting on day 11 to a low dose of 0.125 mg/kg/day by day 17) | 29 | placebo q.i.d. for 30 days |

| | | | | | |
|---|---|---|---|---|---|
| q.i.d. = four times daily | | | | | |

### A Oral Beclomethasone Dipropionate for the Treatment of Patients with Gastrointestinal Graft-Versus-Host Disease (Study No. 615)

This study was an uncontrolled pilot study in 40 GVHD patients, 17 of whom were already taking prednisone for their GVHD at the time of enrollment, and 23 patients who were not (Baehr et al. 1995). Thus, the former group was not responding well to systemic corticosteroids, and the question was whether they would benefit from the addition of a potent, locally acting, corticosteroid administered orally. The question in the latter group was whether such a drug by itself would be adequate to control the gastrointestinal symptoms of GVHD. Both groups of patients received 8 mg/day of oral BDP (2 mg q.i.d.) for 28 days. Each 2 mg dose consisted of one EC (1 mg) and one IR (1 mg) capsule of BDP.

Using the baseline status of patients as their own controls, up to 28 days of BDP resulted in the abatement of most gastrointestinal symptoms and an increase in oral food intake in both groups of patients (i.e. taking prednisone or not). The timing of response in the BDP alone group appeared to be slower than historical experience with similar patients treated with high dose prednisone.

No significant treatment-related adverse events were observed in this study. Study drug associated adverse events reported during the trial included mild metallic taste and mild peri-umbilical heaviness. In the patients not receiving prednisone, tests for HPA axis suppression showed evidence of suppression occurred in the majority of patients. Despite the evidence of some suppression, all of these patients were able to respond at least partially to ACTH administration with an increase in serum cortisol levels. None of these patients developed clinical symptoms of hypocortisolism.

### B Controlled Study of Prednisone With or Without Oral Beclomethasone Dipropionate for the Initial Treatment of Patients with Intestinal Graft-Versus-Host Disease (Study No. 875)

This study was a placebo-controlled phase 2 trial conducted in 60 patients with Grade II GVHD with gastrointestinal symptoms (Study No. 875) (McDonald et al. 1998). In this study, oral BDP was studied as an adjunctive therapy to 10 days of prednisone, i.e. all patients received prednisone and were also randomized to receive active or placebo BDP capsules for 30 days. Oral BDP was administered at 8 mg/day of oral BDP (2 mg q.i.d.). Each 2 mg dose consisted of one EC (1 mg) and one IR (1 mg) capsule of BDP. After day 10, the dose of prednisone was rapidly tapered to a dose of 0.125 mg/kg/day. The primary efficacy endpoint for this study was the proportion of patients in each of the groups who were eating ≥70% of their expected caloric requirements (ECR).

Treatment with oral BDP as an adjuvant to prednisone resulted a significantly higher proportion of patients with increased oral caloric intake compared to treatment with prednisone plus placebo. Gastrointestinal symptoms (nausea, vomiting, diarrhea) all improved in the BDP group compared to the placebo group. BDP was well tolerated, and the incidence of infections between the BDP and placebo groups was similar.

### Examples

A subsequent study was undertaken to assess the effect of a longer term treatment of patients with grade II Graft vs. Host Disease with gastrointestinal symptoms regimen with orally administered BDP in conjunction with ten days of high-dose prednisone therapy.. The primary objective of this multi-center study was to compare the efficacy, defined as time to treatment failure, of an oral BDP regimen (1 mg/kg/day prednisone for 10 days plus 2 mg oral BDP q.i.d. for 50 days) with the efficacy of standard of care (1 mg/kg/day of oral prednisone administered for 10 days plus matching placebo tablets for 50 days) in patients with Grade II graft versus host disease (GVHD) with gastrointestinal (GI) symptoms. The Secondary objectives of the study were:
1. To compare the proportion of treatment failures in the two groups on Study Days 10, 30, 50, 60, and 80.
2. To compare the treatment groups with respect to cumulative systemic corticosteroid exposure.
3. To compare the treatment groups with respect to the incidence and degree of hypothalamic-pituitary-adrenal (HPA) axis suppression in patients who have not experienced treatment failure by Study Day 50.
4. To evaluate the safety of BDP by comparing the treatment groups with respect to treatment-emergent adverse events.
5. To compare the treatment groups with respect to total deaths and cause of death through 200 days post-transplant.
6. To investigate the pharmacokinetic (PK) profile of single and multiple dose administration of 2 mg oral BDP four times daily (split evenly between the immediate release [IR] and the enteric coated [EC] tablets) for 50 days in patients with Grade II GVHD with GI symptoms.

To be eligible for entry into the study, patients were at least 10 days post allogeneic hematopoietic cell transplantation, have GI symptoms consistent with Grade II GVHD, and had endoscopic evidence of GVHD. The diagnosis of GVHD was confirmed by biopsy of the intestine (esophagus, stomach, small intestine, or colon) or skin. A total of 129 eligible patients were randomized to one of two treatment groups.

### Eligibility Criteria

To be eligible to enter the study, patients of any race, age, or gender met the following inclusion criteria:
1. Receipt of an allogeneic hematopoietic cell transplant ≥ 10 days prior to screening.
2. Symptoms consistent with Grade II intestinal GVHD with endoscopic evidence of Grade II GVHD without another plausible etiology (See Table 2).
3. Diagnosis of GVHD confirmed by biopsy of the intestine (esophagus, stomach, small intestine, or colon) or skin (histologic diagnosis within 72 hours prior to the first dose of study drug). Note: if the physician deems that it is inadvisable to do an intestinal biopsy, justification should be stated in the Case Report Form (CRF).
4. Confirmed absence of intestinal infection within 7 days prior to the first dose of study drug.
5. Demonstrated ability to swallow two tablets of the size and configuration of study drug.
6. Anti-candidal prophylaxis of the oropharynx with an effective drug prior to the first dose of study drug.
7. If female and of childbearing potential, must be willing to use adequate contraception, as determined by the investigator, for the duration of the study.
8. Ability to read, understand, and sign (or has legal representative sign) appropriate patient informed consent or assent form.

To be eligible to enter the study, patients did not meet any of the following exclusion criteria:
1. Skin GVHD, other than a slowly evolving rash that involves 50% of the body surface.
2. Liver GVHD with serum bilirubin >3 mg/dL.
3. >1000 mL/day diarrhea on any 1 day within 3 days prior to first dose of study drug.
4. Negative intestinal biopsy for GVHD.
5. Systemic (oral or parenteral) prescription corticosteroid use for the purpose of prophylaxis or treatment of GVHD or another inflammatory disease process within 30 days prior to first dose of study drug. (Note: Patients may continue GVHD prophylaxis with immunosuppressants including cyclosporin, tacrolimus, sirolimus, methotrexate, and mycophenolate mofetil. Use of corticosteroids such as Decadron as an anti-emetic during conditioning therapy, or use of single doses of corticosteroid in conjunction with infusion of blood products or medications to lessen side effects of these infusions, will not exclude patients from study).
6. Persistent vomiting of oral intake that precludes ingestion of study drug tablets.
7. Multi-organ failure, sepsis syndrome, or other condition with high mortality (i.e., life expectancy less than 3 months).
8. Infection of the mouth or esophagus with a fungal organism.
9. Known HIV seropositivity.
10. Pregnancy or lactation.
11. Previous use of BDP tablets, capsules, or inhalation products.
12. Use of any investigational drug, biologic, or device within 30 days prior to first dose of study drug.
13. Inability to comply with the study procedures and scheduled study visits.

### Study Drug

Beclomethasone 17, 21-dipropionate (BDP) is a diester of beclomethasone, a halogenated corticosteroid. For this study, it was supplied and administered in both immediate release (IR) and enteric coated (EC) tablets, both of which consist of the same core formulation containing 1 mg BDP per tablet. Placebo IR and EC tablets were identical in all respects to the BDP IR and EC tablets except for the absence of BDP.

The compositions of the 1 mg IR tablet and of the 1 mg EC tablet are shown in Table 2. The total daily dose was equally divided between the two forms. The rationale for using both dosage forms was to deliver BDP to both the proximal and distal portions of the gastrointestinal tract, with the objective of maximizing the efficacy in treating gastrointestinal inflammation.

**Table 2: Composition of 1 mg BDP Immediate Release Tablets and Enteric Coated Tablets**

| **Component** | **Quantity** |
|---|---|
| **Immediate Release Tablet and Enteric Coated Tablet Core** | |
| Beclomethasone dipropionate, USP | 1.0 |
| Lactose monohydrate, NF | 152.0 |
| Microcrystalline cellulose, NF | 40.0 |
| Croscarmellose sodium, NF | 2.0 |
| Povidone, USP | 4.0 |
| Magnesium stearate, NF | 1.0 |
| | |
| **Enteric Coated Tablet Coating** | |
| Methylacrylic acid copolymer dispersion (Type C), NF | 11.34 |
| Triethyl citrate, NF | 1.7 |
| Polysorbate 80, NF | 0.025 |
| Silicon dioxide, NF | 0.91 |
| Sodium hydroxide, NF | 0.03 |
| | |
| **Total tablet core** | **200.0** |
| **Total tablet coating** | **~14.0** |
| **Total** | **~214.0** |

| | |
|---|---|
| NF = National Formulary; USP = United States Pharmacopoeia | |

### Study Design

This was a multi-center, randomized, double-blind, placebo-controlled, parallel-group study in patients with Grade II GVHD with GI symptoms. To determine eligibility for entry into the study, patients underwent a screening evaluation, including intestinal or skin biopsy and infection work-up.

One hundred twenty-nine patients were enrolled and randomized to receive oral BDP (8 mg/day) or matching placebo tablets for 50 days. The randomization was stratified by the source of the allograft (two HLA haplotype identical sibling versus all other), and by topical corticosteroid use (yes versus no). All patients received 10 days of prednisone at 1 mg/kg/day (or an equivalent dose of intravenous [IV] corticosteroid). On Study Day 10, patients determined by the investigator to have their GVHD controlled had their prednisone tapered rapidly over 7 days starting on Study Day 11, and received a maintenance prednisone dose of 0.0625 mg/kg/day for the remainder of the study. BDP doses were evenly divided to include equal quantities of the IR and EC formulations administered four times per day.

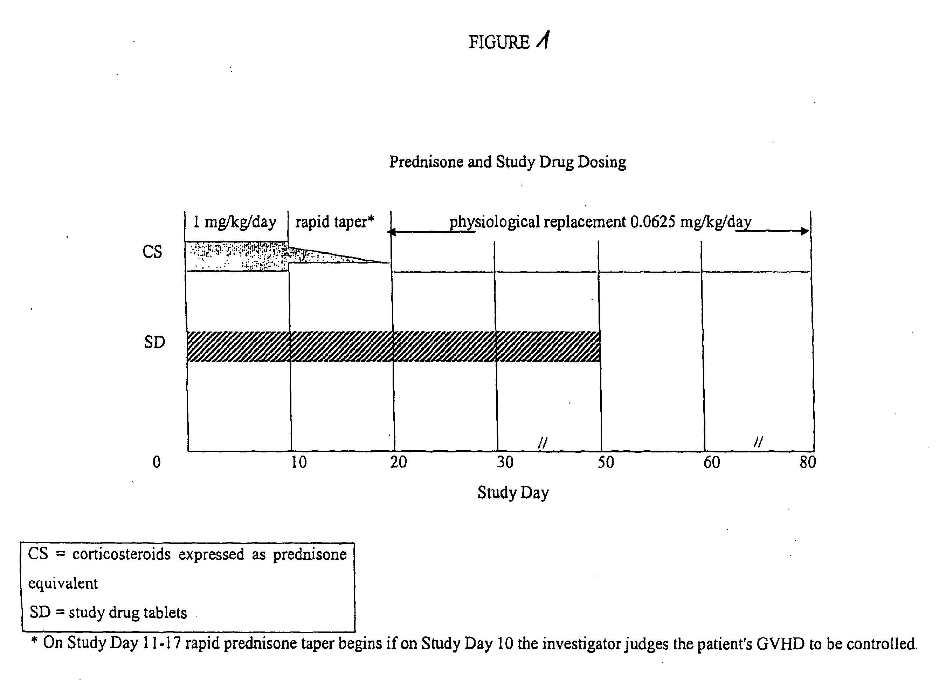

Prednisone and Study Drug Dosing is shown in Figure 1. On Study Day 11-17 rapid prednisone taper begins if on Study Day 10 the investigator judges the patient's GVHD to be controlled.

Screening/baseline evaluations were performed within 3 days prior to the first dose of study drug, except for the confirmed absence of intestinal infection, which may be determined within 7 days. All randomized patients began receiving study drug the morning of Study Day 1, after which some patients will have timed blood samples obtained for PK analysis for up to 24 hours after this dose. For all patients, the second dose of study drug was administered on the morning of Study Day 2, after which dosing was 4 times daily until Study Day 50.

All patients were monitored per standard post transplant routine in the hospital or outpatient clinic. Patients underwent study evaluations, which included blood specimens for PK analysis, on Study Days 10, 30, 50, 60, and 80. In addition, adverse events that occurred during the interval since the prior visit were assessed and recorded. Following the final dose of study drug on the morning of Study Day 50, timed blood samples were obtained for up to 24 hours for PK analysis from some patients. All patients who had normal adrenal responsiveness at Baseline and who reached Study Day 50 without experiencing treatment failure or discontinuing study drug for other reasons returned on Study Day 51 at approximately 8 am for HPA axis function evaluation.

Patients continued their physiological replacement doses of prednisone through Study Day 80. All patients undergo clinic based study evaluations on Study Day 60 and Study Day 80. The Study Day 80 evaluation was conducted by the patient's local physician if the patient had returned home from the transplant center. In the event that a patient experienced significant medical complications and was unlikely to survive the illness, or was entered into hospice care, study drug was discontinued, and the patient did not undergo any further protocol defined study procedures or assessments.

### Study Endpoints

The primary efficacy, safety, and pharmacokinetic endpoints were as follows:

### Efficacy:

1. Treatment failure, defined as use of prednisone or equivalent IV corticosteroids at doses higher than stated in protocol, or addition of other immunosuppressant medications.
2. Kamofsky Scale score if the patient is 16 years of age or older or the Lansky Scale score if the patient is younger than 16 years of age.

### Safety:

1. Adverse events.
2. Cumulative prednisone dose (per kg of body weight).
3. Worsening of GVHD as determined through the assessment of diarrhea, rash, and bilirubin levels.
4. HPA axis function.
5. Hematology measurements (with differential, including eosinophils) at Baseline and Study Day 50.
6. Serum chemistry and urinalysis measurements at Baseline and Study Day 50.
7. Patient survival through day 200 post-transplant.

Pharmacokinetics:
1. Plasma concentrations of BDP and its metabolites, beclomethasone 17-monopropionate (17-BMP) and beclomethasone (BOH) at serial time points following the first and last doses of BDP (Study Days 1 and 50).
2. Trough concentrations of BDP, 17-BMP, and BOH plasma concentrations on Study Days 1, 10, 30, and 50.
3. Maximum plasma concentration (Cₘₐₓ), time to Cₘₐₓ (Tₘₐₓ), area under the curve (AUC), and elimination half-life (t_{1/2}) of BDP, 17-BMP, and BOH after the first (Study Day 1) and last (Study Day 50) doses.

### Statistical Plan

### Efficacy Analyses:

The primary efficacy endpoint of this two-arm study was the time to treatment failure. The primary efficacy analysis will compare the time to treatment failure through Study Day 50 using a log-rank test controlling for patient randomization stratum. Product-limit estimates for time to failure will be provided for descriptive purposes. The null hypothesis for the log-rank test is that time to failure is homogeneous across the treatment groups.

Secondary analyses of treatment failure compared the treatment groups with respect to proportion of treatment failures and Kamofsky Scale (or Lansky Scale) scores at key time points. The difference in proportion of treatment failures on Study Days 10, 30, 50, 60, and 80 was evaluated using a two-sided Fisher's Exact test of the null hypothesis of no difference between the treatment groups. The difference in Karnofsky Scale (or Lansky Scale) scores at the same time points was evaluated using a two-sided Wilcoxon test to evaluate the null hypothesis of no difference in medians between treatment groups.

### Safety Analyses:

Treatment-emergent adverse events was summarized by incidence, incidence within body system, incidence by severity, relatedness to study drug, and outcome. Study drug discontinuation due to adverse event(s) was summarized overall and by days on study.

The ability of BDP to spare GVHD patients the systemic corticosteroid exposure was assessed through the evaluation of cumulative prednisone dose in mg/kg over the course of the study. Descriptive statistics will be used to compare treatment groups.

The worsening of GVHD was evaluated through the assessment of diarrhea (GI), rash (skin), and bilirubin (liver) on Study Days 10, 30, 50, and 60. These endpoints were analyzed using descriptive statistics.

The effect of oral BDP on HPA axis function was assessed by measuring plasma concentrations of adrenocorticotrophic hormone (ACTH), resting morning cortisol, and change in plasma cortisol concentration following a standard test dose of intravenous ACTH. All patients enrolled in the study had their HPA axis function assessed at Baseline (within 2 days prior to Study Day 0). All patients who had normal adrenal responsiveness at Baseline and who reach Study Day 50 without experiencing treatment failure were evaluated for HPA axis suppression on Study Day 51. These patients were on physiologic replacement doses of prednisone, per protocol. A patient was considered to have evidence of abnormal HPA axis function if any of the three tests described above are outside the normal range. The results between and within treatment groups were be analyzed using descriptive statistics.

### Pharmacokinetic Analyses:

BDP, 17-BDP, and BOH plasma concentrations were be measured 12 times in 24 hours on Study Days 1 and 50. If the prevalence and magnitude of plasma concentrations of BDP, 17-BMP, and BOH permitted, then non-compartmental estimates of AUC, Cₘₐₓ, Tₘₐₓ, and t_{1/2} will be computed for each patient randomized to BDP. Paired t-tests will be used to compare the PK parameters on Study Day 1 and 50. Trough concentrations on Study Days 1, 10, 30, and 50 were summarized using descriptive statistics.

### Randomization, Study Drug Treatment Groups, and Prednisone Dosing

Patients were randomized to one of two treatment groups: 8 mg of BDP daily (administered as one 1 mg IR tablet and one 1 mg ER tablet q.i.d.) or placebo tablets. Both regimens included oral prednisone (1 mg/kg/day) or an equivalent dose of IV methylprednisolone (dose of IV methylprednisolone = 0.8 x dose of oral prednisone) given in 2 divided doses per day on Study Days 1 to 10.

Eligible patients fit into one of four strata: 1) two HLA haplotype identical sibling with topical corticosteroid use at baseline; 2) other allograft source with topical steroid use at baseline; 3) two HLA haplotype identical sibling graft with no topical corticosteroid use at baseline; or 4) other allograft source with no topical steroid use at baseline. After determining the correct stratum for a patient, the pharmacist assigned the next patient number in that stratum to the patient. The pharmacist will dispense the study drug according to a block randomization schedule unique to each site that is provided by Enteron.

On Study Day 10, patients were evaluated for the signs and symptoms of GVHD. Patients in both treatment groups who met the response criterion of adequately controlled GVHD, as judged by the investigator, had their prednisone tapered over 7 days starting on Study Day 11 (Table 3). Starting on Study Day 17, physiologic replacement doses of prednisone were given for the remainder of the study. If the investigator determined that GVHD was not controlled on Study Day 10, patients discontinued study drug but continued prednisone initially at 1 mg/kg/day; these patients were considered to be treatment failures. In treatment failure cases, physicians subsequently modifiedy prednisone dosage as deemed appropriate.

**Table 3: Prednisone Dose and Taper Schedule**

| **Study Days** | **Prednisone Dosing Schedule*** | **Total Daily Prednisone Dose*** |
|---|---|---|
| 1-10 | 0.5 mg/kg b.i.d. | 1.0 mg/kg |
| 11-12 | 0.25 mg/kg b.i.d. | 0.5 mg/kg |
| 13-14 | 0.125 mg/kg b.i.d. | 0.25 mg/kg |
| 15-16 | 0.0625 mg/kg b.i.d. | 0.125 mg/kg |
| 17-80 | 0.0625 mg/kg q.d. | 0.0625 mg/kg |

| | | |
|---|---|---|
| *Or an equivalent dose of IV corticosteroid (dose of IV methylprednisolone = 0.8 x dose of oral prednisone) | | |

Prednisone dosing calculations throughout the study were be based on the weight of the patient at the Baseline Visit (Study Day 0). The Daily Diary will be used to collect daily prednisone dosing information.

If signs or symptoms of adrenal insufficiency occurred during tapering, prednisone was resumed at higher doses, depending on the level of medical stress, and a more gradual taper implemented. If a patient's GVHD worsened during the tapering of prednisone, higher doses of prednisone were resumed at the discretion of the investigator, and the patient were considered a treatment failure. However, an exception were made to this definition of treatment failure if the higher dose of corticosteroids was prescribed for

### <96 hours to cover the possibility of adrenal hyporesponsiveness during an anticipated period of medical stress, such as surgery.

### Results

**DOR BioPharma, Inc. Announces Top-Line Results of Pivotal Phase III Clinical Trial of orBec® for Intestinal Graft-versus-Host Disease or Bec® Demonstrates Positive Trend in Primary Endpoint and a Highly Statistically Significant Reduction in Mortality**

Miami, FL, December 30, 2004 - DOR BioPharma, Inc. (AMEX: DOR) ("DOR" or the "Company") announced the top-line results of its multi-center, pivotal Phase III clinical trial of or Bec® (oral beclomethasone dipropionate) for the treatment of intestinal Graft-versus-Host Disease (iGVHD).

### About Intestinal Graft-versus-Host Disease (iGVHD)

iGVHD is a life threatening condition that is one of the most common causes for the failure of bone marrow transplant procedures. These procedures are being increasingly utilized to treat leukemia and other cancer patients with the prospect of eliminating residual disease and reducing the likelihood of relapse. or Bec® represents a first-of-its-kind oral, locally acting therapy tailored to treat the intestinal manifestation of GVHD, the organ system where GVHD is most frequently encountered and highly problematic. or Bec® is intended to reduce the need for systemic immunosuppressives to treat iGVHD. Currently approved systemic immunosuppressives utilized to control iGVHD substantially inhibit the highly desirable graft-versus-leukemia (GVL) effect of bone marrow transplants, leading to high rates of aggressive forms of relapse, as well as substantial rates of mortality due to opportunistic infection.

### Study Design

One hundred and twenty-nine (129) post-bone marrow transplant patients presenting with Grade II iGVHD were enrolled in the randomized, double-blind, placebo-controlled, multi-center clinical trial, which was conducted at 16 bone marrow transplant centers in the United States and France. All patients in the Phase III clinical trial were initially treated with a constant daily dose of high dose (1-2 mg/kg) prednisone, which is the current standard therapy, in combination with an oral dose of either orBec® (8mg/day) or placebo for the first 10 days. On day 10, if patients were responding to treatment, the high dose prednisone was rapidly tapered and patients continued to receive either orBec® or placebo orally for an additional forty days. The primary endpoint of the study was a comparison between the two treatment arms of the time to treatment failure, defined as the need for additional therapies due to uncontrolled signs or symptoms of GVHD. Secondary endpoints in the study included a comparison of the proportion of treatment failures and clinical scores at 10 Days, 30 Days, 50 Days, 60 Days and 80 Days post-randomization, a comparison of cumulative exposure to systemic steroids, as well as a comparison of mortality at Day 200 post-transplant (Table 4)

**Table 4:**

| **Current Top-Line Data** | | | |
|---|---|---|---|
| EndPoints | orBec® n=62 | Placebo n=67 | p-value orBec® vs. placebo |
| Time to Treatment Failure at Study Day 50 (primary endpoint) | n/a | n/a | 0.1177 |
| Treatment Failure Rate at Study Day 50 | 31% | 48% | 0.0515 |
| Time to Treatment Failure at Study Day 80 | n/a | 52 | 0.0226 |
| Treatment Failure Rate at Study Day 80 | 39% | 65% | 0.0048 |
| Mortality Rate at 200 Days Post-Transplant | 5 (8%) | 17 (26%) | 0.006 |

| | | | |
|---|---|---|---|
| n/a = not achieved due to statistical methodology | | | |

### Time to Treatment Failure

While orBec® did not achieve statistical significance in its primary endpoint of time to treatment failure through Day 50 (p-value 0.1177), orBec® did achieve statistical significance in its secondary endpoint of time to treatment failure through Day 80 (p-value 0.0226). The Company believes that the p-value of 0.1177 achieved in the primary endpoint through Day 50 is primarily due to a higher than expected rate of treatment failures during days 0-10 of the study. During such period, patients were receiving high dose prednisone (1-2mg/kg/day) plus either orBec® (8mg/day) or placebo. For purposes of the study, patients that did not begin the rapid taper of high dose prednisone on Day 10 as called for by the regimen were deemed treatment failures for all purposes, including the calculation of statistical significance of time to treatment failure at Day 50. The Company intends to further analyze the Day 0-10 treatment failure group and the statistical impact of this group on the primary endpoint of time to treatment failure at Day 50 and discuss the results of this analysis with the FDA. Encouragingly, the treatment failure rate at Day 50 approached statistical significance (p-value 0.0515). In addition, the secondary endpoint of time to treatment failure at Day 80, as well the treatment failure rate at Day 80, each achieved statistical significance (p-values 0.0226 and 0.0048, respectively) (Table 4 and 5).

### orBec® Demonstrates Highly Statistically Significant Reduction in Mortality

Perhaps of greatest clinical relevance, orBec® demonstrated a 70% reduction in mortality, registering only 5 (8%) deaths during the prospectively defined Day 200 post-transplant period versus 17 (26%) deaths for the placebo group (p-value 0.006). Based upon separate analysis conducted by the Company, there is also a statistically significant correlation between treatment failure and mortality (Table 6).

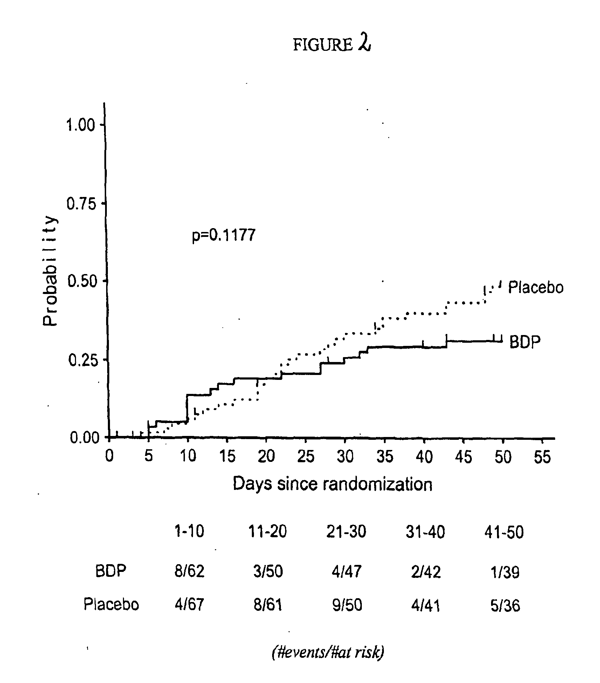

Figure 2 shows the cumulative treatment failures during the first 50 days in patients receiving placebo or BDP.

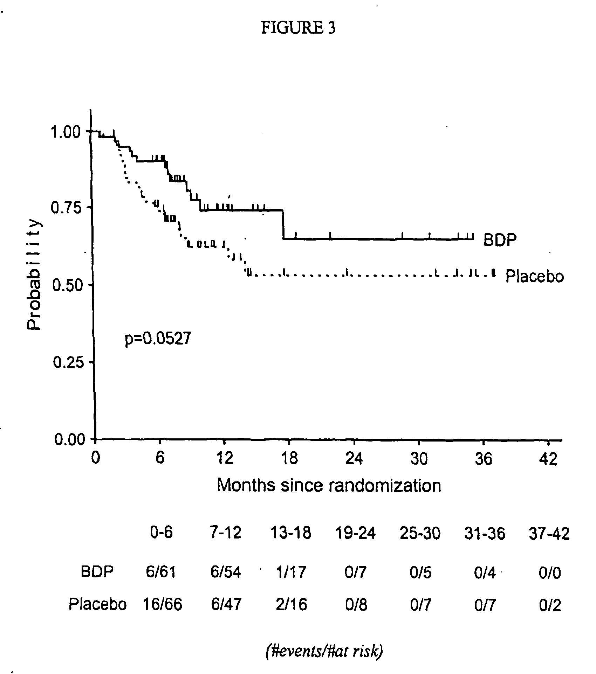

Figure 3 shows the duration of overall survival-post randomization (safety population).

**Table 5. Cumulative Treatment Failure Rate at Specified Time Points Estimates Based on Kaplan-Meier Method (Full Analysis Set)**

| | Placebo | BDP |
|---|---|---|
| Subjects randomized | 67 | 62 |
| Study day 10 | | |
| Cumulative events | 4 | 8 |
| Treatment failure rate (95% CI) | 0.06 (0.03, 0.13) | 0.14 (0:08, 0.24) |
| | Z = 1.426, P = 0.1538 | |
| Study day 30 Cumulative events | 21 | 15 |
| Treatment failure rate (95% CI) | 0.33 (0.25, 0.45) | 0.25 (0.18, 0.37) |
| | Z = 0.911, P = 0.3625 | |
| Study day 50 | | |
| Cumulative events | 30 | 18 |
| Treatment failure rate (95% CI) | 0.48 (0.39, 0.60) | 0.31 (0.23, 0.43) |
| | Z = 1.948, P = 0.0515 | |
| Study day 60 | | |
| Cumulative events | 31 | 19 |
| Treatment failure rate (95% CI) | 0.50 (0.41, 0.62) | 0.33 (0.24, 0.45) |
| | Z = 1.913, P = 0.0558 | |
| Study day 80 | | |
| Cumulative events | 39 | 22 |
| Treatment failure rate (95% CI) | 0.65 (0.55,0.76) | 0.39 (0.30, 0.52) |
| | Z = 2.819, P = 0.0048 | |

The cumulative treatment failure rate is estimated from the complement of the Kaplan-Meier distribution.

The variance (standard error) of the Kaplan-Meier estimate is calculated using Greenwood's formula.

Test of difference in treatment failure rates (Placebo - BDP) based on the Z-test. Significance level of 0.05 (two-sided). No adjustments for multiple tests of significance.

**Table 6. Overall Survival Status - 200 Days Post-Transplant (Safety Population)**

| | Placebo | | BDP | | Overall | |
|---|---|---|---|---|---|---|
| Subjects randomized | 67 | | 62 | | 129 | |
| Subjects evaluable for safety* | 66 | | 61 | | 127 | |
| Survival status | | | | | | |
| Alive | 49 | 74% | 56 | 92% | 105 | 83% |
| Dead | 17 | 26% | 5 | 8% | 22 | 17% |
| Day 200 survival rate (95% CI) | 0.74 (0.66, | | 0.91 | (0.86, | 0.83 | (0.77, |
| | Z = | 2.750, P = | 0.0060 | | | |

## Claims

1. Beclomethasone dipropionate (BDP) together with prednisone for use in the reduction of mortality associated with graft-versus-host disease resulting from hematopoietic cell transplantation, by administration to a subject in need thereof a) about 1-2 mg/kg/day prednisone for about 10 days, tapering over the following 7 days to a physiological replacement dose of about 0.0625 mg/kg/day for the remainder of the treatment, and b) about 4-12 mg oral BDP q.i.d. for about 50 days, wherein the BDP is administered in both immediate release and enteric coated preparations.

2. The combination for use according to claim 1, wherein said subject is human.

3. The combination for use according to claim 1, wherein the BDP is administered orally at a dosage or between about 4 mg/day to about 8 mg/day or at a dosage of between about 2 mg/day to about 4 mg/day.

4. The combination for use according to claim 1, wherein the BDP is in the form of a pill, tablet, capsule or microsphere.

5. The combination for use according to claim 4, wherein the BDP is formulated such that the pill, microsphere, or capsule dissolves in the stomach, small intestine or colon.

6. The combination for use according to claim 1, wherein BDP is formulated for oral administration in the form of an emulsion.

7. The combination for use according to claim 1, wherein administration of the BDP ceases 50 days following the beginning of said co-administration therapy.

8. The combination for use according to claim 1, wherein the patient has received an allogeneic bone marrow transplant or an allogeneic blood transplant.

## Patentansprüche

1. Beclomethasondipropionat (BDP) zusammen mit Prednison zur Verwendung zur Verringerung der mit der Graft-versus-host-Krankheit assoziierten Sterblichkeit, die durch eine hämatopoietische Zelltransplantation verursacht wird, durch Verabreichen an ein Subjekt das dies benötigt von (a) ungefähr 1-2 mg/kg/Tag Prednison für ungefähr 10 Tage, Reduzieren im Verlauf der folgenden 7 Tage auf eine physiologische Ersatzdosis von 0,0625 mg/kg/Tag für den Rest der Behandlung, und von (b) ungefähr 4-12 mg oralem BDP q.i.d. für ungefähr 50 Tage, wobei das BDP sowohl in Zubereitungen mit sofortiger Freigabe als auch in Zubereitungen mit enterischem Überzug verabreicht wird.

2. Kombination zur Verwendung nach Anspruch 1, wobei das Subjekt ein Mensch ist.

3. Kombination zur Verwendung nach Anspruch 1, wobei das BDP oral in einer Dosierung von ungefähr 4 mg/Tag bis ungefähr 8 mg/Tag oder in einer Dosierung von ungefähr 2 mg/Tag bis ungefähr 4 mg/Tag verabreicht wird.

4. Kombination zur Verwendung nach Anspruch 1, wobei das BDP in der Form einer Pille, Tablette, Kapsel oder Mikrosphäre vorliegt.

5. Kombination zur Verwendung nach Anspruch 4, wobei das BDP so formuliert ist, dass sich die Pille, Mikrosphäre oder Kapsel im Magen, Dünndarm oder Dickdarm auflösen.

6. Kombination zur Verwendung nach Anspruch 1, wobei das BDP für die orale Verabreichung in Form einer Emulsion formuliert ist.

7. Kombination zur Verwendung nach Anspruch 1, wobei die Verabreichung von BDP 50 Tage nach dem Beginn der Co-Verabreichungstherapie eingestellt wird.

8. Kombination zur Verwendung nach Anspruch 1, wobei der Patient eine allogene Knochenmarkstransplantation oder eine allogene Bluttransplantation erhalten hat.

## Revendications

1. Dipropionate de béclométhasone (BDP) conjointement avec de la prednisone pour une utilisation dans la réduction de la mortalité associée à la maladie du greffon contre l'hôte résultant d'une transplantation de cellules hématopoïétiques, par l'administration à un sujet en ayant besoin de
a) environ 1 à 2 mg/kg/jour de prednisone pendant environ 10 jours, en diminuant sur les 7 jours suivants jusqu'à une dose de remplacement physiologique d'environ 0,0625 mg/kg/jour pour le reste du traitement, et
b) environ 4 à 12 mg de BDP oral quatre fois par jour pendant environ 50 jours, où le BDP est administré dans des préparations à la fois à libération immédiate et gastro-résistantes.

2. Combinaison à utiliser selon la revendication 1, où ledit sujet est un être humain.

3. Combinaison à utiliser selon la revendication 1, où le BDP est administré par voie orale à une posologie ou entre environ 4 mg/jour et environ 8 mg/jour ou à une posologie entre environ 2 mg/jour et environ 4 mg/jour.

4. Combinaison à utiliser selon la revendication 1, où le BDP est sous la forme d'une pilule, d'un comprimé, d'une capsule ou d'une microsphère.

5. Combinaison à utiliser selon la revendication 4, où le BDP est formulé de telle manière que la pilule, la microsphère ou la capsule se dissout dans l'estomac, l'intestin grêle ou le côlon.

6. Combinaison à utiliser selon la revendication 1, où le BDP est formulé pour une administration orale sous la forme d'une émulsion.

7. Combinaison à utiliser selon la revendication 1, où l'administration du BDP cesse 50 jours après le début de ladite thérapie par coadministration.

8. Combinaison à utiliser selon la revendication 1, où le patient a reçu un transplant de moelle osseuse allogénique ou un transplant de sang allogénique.
